# EUROPEAN PATENT SPECIFICATION

(11) **EP 1 614 430 B1**
(45) Date of publication and mention of the grant of the patent: **07.09.2016**
(21) Application number: 05254286.7
(22) Date of filing: 07.07.2005
(51) Int. Cl.: A61K 45/06, A61P 17/12, A61P 17/10, A61Q 19/00, A61Q 19/02, A61K 8/368, A61K 8/37, A61K 8/42, A61K 31/60

(54) **Compositions containing anti-acne agents and the use thereof**
Zusammensetzungen, die Anti-Aknewirkstoffe enthalten und deren Verwendung
Compositions contenant des agents anti-acneiques et leur utilisations

(30) Priority: 07.07.2004 US 886314; 20.06.2005 US 156404
(43) Date of publication of application: 11.01.2006
(73) Proprietor: Johnson & Johnson Consumer Inc., Skillman, NJ 08558 (US)
(72) Inventor: Wu, Jeffrey M., Warrington, PA 18976 (US); Liu, Jue-Chen, Belle Mead, NJ 08502 (US); Chantalat, Jeannette, Princeton, NJ 08540 (US); Sun, Ying, Belle Mead, NJ 08502 (US); Johnsen, Stefanie A., Piscataway, NJ 08854 (US); Jampani, Hanuman B., Flemington, NJ 08822 (US)
(74) Representative: Kirsch, Susan Edith

(56) References cited:
- WO-A-00/06116
- WO-A-98/33471
- WO-A1-03/075883
- GB-A- 1 470 355
- US-A- 4 540 567
- US-A- 5 652 266
- US-A1- 2002 114 820
- US-B1- 6 248 343

## Description

### BACKGROUND OF THE INVENTION

Acne is a skin disorder that is often accompanied by pimples that sometimes cause acne sufferers embarrassment. Consumers have utilized topical anti-acne agents, such as salicylic acid and benzoyl peroxide to treat acne for many years. Topical anti-acne agents typically function by exfoliating skin and/or killing bacteria on the surface of the skin. Although such anti-acne agents are often effective at treating acne pimples, they tend to take a long time, typically days or weeks, to abate acne symptoms. They are also often ineffective at treating pre-emergent pimples.

Pigment abnormalities such as post-inflammatory hyperpigmentation (PIH) and scars are often caused by acne, pseudofolliculitis barbae (PFB), and other follicular diseases. It is more common in darker skin types, but all skin types can suffer from these post-inflammatory marks. The presence of PIH and dark marks results in skin that does not appear smooth, clear, nor even.

GB 1 470 355 A discloses an anti-acne composition for topical administration comprising from 0.01 to 0.2% by weight of at least one of 1,1'-hexamethylenebis-5-(hexyl)biguanide, 1,1'-hexamethylenebis-5-(heptyl)biguanide, 1,1'-hexamethylenebis-5-(n-octyl)biguanide, 1,1'-hexamethylenebis-5-(2-ethylhexyl)biguanide and 1,1 '-hexamethylenebis-5-(nonyl)biguanide and their pharmaceutically acceptable acid-addition salts as an anti-acne agent; at least one of lauryl lactate, methyl pyrrolidone, methyl salicylate and Vitamin A acid as a skin-penetrating agent, in which the lauryl lactate constitutes from 10 to 20% by weight of the formulation, the methyl pyrrolidone constitutes from 36 to 41% by weight of the formulation, the methyl salicylate constitutes from 5 to 9% by weight of the formulation and the Vitamin A acid constitutes from 0.05 to 0.1% by weight of the formulation; and a pharmaceutically acceptable solvent suitable for topical application. The composition may further comprise 0.01-0.15% by weight of bactylated hydroxy toluene, butylated hydroxy anisole and/or ascorbic acid as anti-excipient and may be made up as a lotion, freshener, cream, ointment, jelly, salve or emulsion.

US 5,652,266 A discloses a cosmetic or dermatological composition containing a skin enhancing agent consisting essentially of a) at least one alpha-hydroxy acid, b) salicylic acid or esters thereof and c) at least one retinoid in a skin enhancing method.

WO 03/075883 A1 discloses a topical skin care composition containing a retinoid and a non-paraben preservative and being substantially free of paraben preservatives.

US 4,540,567 A discloses a cosmetically acceptable composition containing a C1-C4 alkyl lactate dissolved in a mixture of water and a water-miscible C2-C4 alkylene glycol of a polymer thereof. The composition has a stabilized pH of less than 7.

### SUMMARY OF THE INVENTION

The present invention features a composition including an anti-acne agent, an antimicrobial agent, and a lactate as described in claim 1. Also described is a method of treating a follicular disease, such as acne, by applying a composition to an area of skin in need of such treatment. Also described is a method of reducing the appearance of pores or oil on the skin by applying a composition to an area of skin in need of such treatment. Also described is a method of evening skin tone or smoothing skin by applying a composition to an area of skin in need of such treatment.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Whenever used, any percentage is weight by weight (w/w) unless otherwise indicated.

As used herein, "topically applying" means directly laying on or spreading on outer skin, e.g., by use of the hands or an applicator such as a wipe, puff, roller, or spray. As used herein, "cosmetically-acceptable" means that the compound(s) or composition(s) which the term describes are suitable for use in contact with tissues (e.g., the skin) without undue toxicity, incompatibility, instability, irritation, allergic response, and the like. This term is not intended to limit the compound/composition to which it describes for use solely as a cosmetic (e.g., the ingredient/product may be used as a pharmaceutical).

As used herein, "safe and effective amount" means an amount of compound(s) or composition(s) sufficient to treat acne, but low enough to avoid serious side effects.

What is meant by "promoting" is promoting, advertising, or marketing. Examples of promoting include, but are not limited to, written, visual, or verbal statements made on the product containing the composition or in stores, magazines, newspaper, radio, television, internet, and the like.

The compositions of the present invention are useful for treating follicular diseases, such as acne, rosacea, hyperlipidemia, seborrhea, sebacious hyperplasia, follicular rash, demodex folliculorum follicular infections such as folliculitis, staphylcoccal impetigoacne necrotica, and psudofolliculitis barbe, follicular ketarosis, keratosis pilaris, phrynoderma, ichthyosis follicularis, alopecia, follicular dysplasia, hirsutism, oily skin, and hypertrichosis. As used herein, the term "treating" or "treatment" means the treatment (e.g., alleviation or elimination of symptoms and/or cure) and/or prevention or inhibition of the condition (e.g., a skin condition).

The compositions of the present invention are also useful for evening skin tone (such as lightening dark areas of skin) in need of such treatment, smoothing the skin (such as reducing texture on the skin), reducing the production of sebum, and reducing the appearance of oil, shine, and/or pores on skin in need of such treatment. Examples of such skin in need of such treatment include, skin having excessive pigmentation (such as freckles, post-inflammatory hyperpigmentation (PIH), or pigmented scars), rough skin, oily skin, or skin having large, visible pores.

In one embodiment, the composition is for the treatment of acne, including the treatment or prevention of acne blemishes, acne pimples, pre-emergent pimples, blackheads, and/or whiteheads. What is meant by a "pre-emergent pimple" is an inflamed follicle that are not visually apparent on the surface of the skin with the naked eye (e.g., as a lesion). In one embodiment, the appearance of acne (e.g., the size and/or appearance of the acne lesion and/or blackhead) is reduced within about eight hours, such as within about four hours.

The present invention relates to topical compositions including an anti-acne agent as described in claim 1. What is meant by an anti-acne agent is an compound that has been approved by the U.S. Food and Drug Administration for the topical treatment of acne. The anti-acne agent is selected from the group consisting of salicylic acid. In one embodiment, the amount of anti-acne agent in the composition is from 0.01% to 10%, for example from 0.1 % to 5%, or from 0.5% to 2% by weight, based on the total weight of the composition.

In one embodiment, the compositions of the present invention include a hair growth agent. What is meant by a hair growth agent is a compound that induces hair growth. Examples of hair growth agents include, minoxadil, spironolactone, cyproterone acetate, azelaic acid, buserelin, bicalutamide, cromakalim, cyclosporin, aminoglutethimide, cyproterone acetate, diazoxide, phenytoin, estradiols, flutamide, prezatide copper, inocoterone, leuprolide acetate, ketoconazole, pinacidil, progesterone, finasteride, retinoic acid, turosteride, vitamins and minerals such as vitamin E, niacin (vitamin B3), pantothenic acid (vitamin B5), pyridoxine (vitamin B6), vitamin B12, vitamin C, vitamin K, biotin, inositol, zinc, copper, cysteine, methionine, coenzyme Q10, essential fatty acids such as flaxseed oil, primrose oil, and fish oil, herbal extracts such as ginko biloba, and combinations thereof.

In one embodiment, the compositions of the present invention include a hair retardation agent. What is meant by a hair retardation agent is a compound that reduces the appearance and/or growth of hair. Examples of hair retardation agents include, eflornithine hydrochloride, soy extracts, antiandrogenic sterols from serenoa (Serenoa repens) and/or from Cucurbita seeds (Cucurbita pepo), dipeptide N-(Carboxymethyl)phenylalanyl-β-alanine compounds, 3-deazaneplanocin, inhibitors of nitric oxide synthetase such as NG-methyl-L-arginine, inhibitors of glutamine metabolism such as 6-diazo-5-oxonorleucine (I), and combinations thereof.

The compositions of the present invention include an antimicrobial agent as defined in claim 1. What is meant by an antimicrobial agent is a compound that kills microorganisms or prevents or inhibits their growth or reproduction.

In one embodiment, the amount of antimicrobial agent in the compositions is from 0.001% to 10%, such as from 0.01% to 5% such as from 0.05% to 2% by weight, based on the total weight of the composition.

In one embodiment, the compositions of the present invention include an antipsoriatic agent. Examples of antipsoriatic agents include, corticosteroids (e.g., betamethasone dipropionate, betamethasone valerate, clobetasol propionate, diflorasone diacetate, halobetasol propionate, triamcinonide, dexamethasone, fluocinonide, fluocinolone acetonide, halcinonide, triamcinolone acetate, hydrocortisone, hydrocortisone verlerate, hydrocortisone butyrate, aclometasone dipropionte, flurandrenolide, mometasone furoate, and methylprednisolone acetate), methotrexate, cyclosporine, calcipotriene, anthraline, shale oil, elubiol, ketoconazole, coal tar, salicylic acid, zinc pyrithione, selenium sulfide, hydrocortisone, sulfur, menthol, and pramoxine hydrochloride, and combinations thereof.

In one embodiment, the compositions of the present invention include an anti-viral agent. Examples of anti-viral agents include, imiquimod, podofilox, podophyllin, interferon alpha, acyclovir, famcyclovir, valcyclovir, reticulos and cidofovir. In one embodiment, the compositions of the present invention include an anti-inflammatory agent. Examples of anti-inflammatory agents, include, non-steroidal and steroidal anti-inflammatory agents such as corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, fluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, and triamcinolone, and combinations thereof. Examples of non-steroidal anti-inflammatory agents include COX inhibitors, LOX inhibitors, and p38 kinase inhibitors, immunosuppresant agents such as cyclosporin, and cytokine synthesis inhibitors. Other natural anti-inflammatories include, extracts of feverfew, soy, or oats, beta-glucan, and totarol.

Other active agents include wound healing enhancing agents such as calcium alginate, collagen, recombinant human platelet-derived growth factor (PDGF) and other growth factors, ketanserin, iloprost, prostaglandin E1 and hyaluronic acid; scar reducing agents such as mannose-6-phosphate; analgesic agents; debriding agents such as papain, and enzymatic debriding agents; and anesthetics such as lidocaine and benzocaine. In one embodiment, the composition comprises one or more of menthol, camphor, an antihistamine, or a local anesthetic such as tetracaine, lidocaine, prilocaine, benzocaine, bupivacaine, mepivacaine, dibucaine, etidocaine, butacaine, cyclomethycaine, hexylcaine, proparacaine, and lopivacaine, capsaicin, or oatmeal.

In one embodiment, the amount of anti-inflammatory agent, anti-viral agent, antipsoriatic agent and/or other active agent in the compositions is from 0.001% to 10%, such as from 0.01% to 5% such as from 0.05% to 2% by weight, based on the total weight of the composition.

The compositions of the present invention further includes a lactate as described in claim 1. The lactates are selected from the group consisting of cetyl lactate and C12-C15 lactates. The amount of lactates in the composition of the present invention may vary from 0.1% to 50%, for example from 0.5% to 20%, or from 1% to 10% by weight, based on the total weight of the composition.
In one embodiment, the composition of the present invention further comprises a phospholipid. Examples of phospolipids include, synthetic phospholipids and natural phospholipids such as phospholipids composed of diester and triester phosphatides such as Cocamidopropyl PG-Dimonium Chloride (Colalipid C™, Colonial Chemical, Inc., South Pittsburgh, TN, USA), Stearamidopropyl PG-Dimonium Chloride (Colalipid ST™), Sunfloweramidopropyl phosphate PG-dimonium chloride (Colalipid SUN™), Sodium Olivamidopropyl PG-Dimonium Chloride Phosphate(Colalipid OL™), Sodiumgrapeseedamindopropyl PG-Dimonium Chloride Phosphate (Colalipid GS™), Linoleamidopropyl PG-Dimonium Chloride Phophate (Colalipid SAFEL™), PEG-8 dimethicone sunfloweramidopropyl PG-dimonium complex (Colalipid SIL™), ricinoleamidopropyl PG-dimonium chloride phosphate (Colalipid RC™), sodium coco PG-dimonium chloride phosphate (Arlasilk™ phospholipids CDM, Uniqema, ICI Group of Companies, Wilton, UK), Cocamidopropyl PG-Dimonium Chloride (ArlasilkTM phospholipids PTC), stearamidipropyl PG-dimonium chloride phosphate (ArlasilkTM phospholipids SV), linoleamidopropyl PG-dimonium chloride phosphate (ArlasilkTM phospholipids EFA), linoleamidopropyl PG-dimonium chloride phosphate dimethicone(ArlasilkTM phospholipids PLN), Myristamidopropyl PG-Dimonium Chloride Phosphate (ArlasilkTM phospholipids PTM), and sodium borageamidopropyl PG-dimonium chloride phosphate (ArlasilkTM phospholipids GLA), and combinations thereof.
In one embodiment, the composition includes a sebum miscible agent. What is meant by a sebum miscible agent is an agent that is miscible with sebum as set forth in the following assay. Artificial sebum is prepared as set forth on page 79 (Table 5.4) of a book chapter entitled "The Influence of Skin Surface Lipids on Topical Formulations" by Obsorne and Hatzenbuhler (in "Topical Drug Delivery Formulations", edited by D. Osborne and A. Amann, Marcel Dekker, Inc., New York, 1990, pages 69-85). At room temperature this sebum is a white waxy substance. 50 µl of the sebum is deposited into a 200 µl clear vial using a precision micropipette. 100 µl of the test agent is then added to the vial. The vial was warmed at 32oC and visually inspected at the baseline and at eight hours. If the agent is miscible with the sebum, the sebum will become transparent.
The following is a non-limiting example of sebum miscible agents: aromatic alcohols such as phenyl alcohols with chemical structures of C6H5-R(OH) where R is an aliphatic radical, such as benzyl alcohol and phenethyl alcohol; aromatic glycol ethers such as ethylene glycol phenyl ether; propylene or butylene oxide-based glycol ethers such as propylene glycol methyl ether and those disclosed in U.S. Patent No. 5,133,967; fatty acids, polyunsaturated fatty acids such as linoleic acid, linolenic acid, stearidonic acid, plant, fruit, or marine derived extracts rich in essential fatty acid or polyunsaturated fatty acids such as but not limited to vaccinium myrtillus (bilberry) seed oil, vaccinium macrocarpon (cranberry) seed oil, vaccinium vitis-idaea (lingonberry) seed oil, rubus idaeus (raspberry) seed oil, rubus chamaemorus (cloudberry) seed oil, ribes nigrum (black currant) seed oil, hippophae rhamnoides (sea buckthorn) seed oil, echium plantagineum (echium) seed oil, hordeum vulgare (barley) seed oil, betula alba bud extract, saw palmetto extract, borage oil, evening primrose oil, witch hazel extract and soy oil; cetyl ocenate; isostearyl benzoate; pentaerythiol teraoctenate; isostearyl benzoate; methyl gluceth; tocopherol acetate; benzalkonium chloride; and benzethonium chloride, and combinations thereof.

The compositions of the present invention may further include an alcohol. Examples of suitable alcohols include, ethyl alcohol. In one embodiment, the composition includes less than 40%, such as from 0.01% to 40%, for example from 0.1% to 30%, or from 1% to 20% by weight, of alcohol based on the total weight of the composition.

In one embodiment, the composition includes a nonionic surfactant. Examples of nonionic surfactants are disclosed on pages 2955-2976 of the International Cosmetic Ingredient Dictionary and Handbook, eds. Wenninger and McEwen, (The Cosmetic, Toiletry, and Fragrance Assoc., Washington, D.C., 9th Edition, 2002) (hereinafter "CTFA Handbook").

In one embodiment, the composition includes a skin lightening agent. Examples of skin lightening agents include, retinoids such as retinol, extracts of soy or licorice, and tyrosine inhibitors such as hydroquinone, ascorbyl glucoside, kojic acid, calcium D-pantetheine-S-sulfonate, arbutin, magnesium ascorbyl phosphate, pantothiol, dihydrolipoic acid, and arlatone.

In one embodiment, the composition of the present invention has a pH greater than 2 and a pH less than 10 such as less than 7, such as less than 4.5.

The topical compositions useful in the present invention involve formulations suitable for topical application to skin. The composition may further include a cosmetically-acceptable topical carrier. The cosmetically-acceptable topical carrier may comprise from 50% to 99%, by weight, of the composition (e.g., from 80% to 95%, by weight, of the composition).
The compositions may be made into a wide variety of product types that include solid and liquid compositions such as lotions, creams, gels, sticks, sprays, shaving creams, ointments, cleansing liquid washes and solid bars, pastes, powders, mousses, masks, peels, make-ups, and wipes. These product types may comprise several types of cosmetically acceptable topical carriers including, solutions, emulsions (e.g., microemulsions and nanoemulsions), gels, solids and liposomes). The compositions may be used in conjunction with other devices such as skin abrading, skin messaging, electro-stimulation devices, light-therapy devices, ultrasound devices, radio frequency devices, thermal/cooling devices, and micro-penetration devices. The following are non-limitative examples of such carriers. Other carriers can be formulated by those of ordinary skill in the art.

The topical compositions useful in the present invention can be formulated as solutions. Solutions typically include an aqueous solvent (e.g., from 50% to 99% or from 90% to 95% of a cosmetically acceptable aqueous solvent).
Topical compositions useful in the subject invention may be formulated as a solution comprising an emollient. Such compositions preferably contain from 2% to 50% of an emollient(s). As used herein, "emollients" refer to materials used for the prevention or relief of dryness, as well as for the protection of the skin. A wide variety of suitable emollients are known and may be used herein. See International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "CTFA Handbook") contains numerous examples of suitable materials.
A lotion can be made from such a solution. Lotions typically comprise from 1% to 20% (e.g., from 5% to 10%) of an emollient(s) and from 50% to 90% (e.g., from 60% to 80%) of water.
Another type of product that may be formulated from a solution is a cream. A cream typically comprises from 5% to 50% (e.g., from 10% to 20%) of an emollient(s) and from 45% to 85% (e.g., from 50% to 75%) of water.
Yet another type of product that may be formulated from a solution is an ointment. An ointment may comprise a simple base of animal or vegetable oils or semi-solid hydrocarbons. An ointment may comprise from 2% to 10% of an emollient(s) plus from 0.1% to 2% of a thickening agent(s). A more complete disclosure of thickening agents or viscosity increasing agents useful herein can be found in the CTFA Handbook pp. 1693-1697.
The topical compositions useful in the present invention may be formulated as emulsions. If the carrier is an emulsion, from 1% to 10% (e.g., from 2% to 5%) of the carrier comprises an emulsifier(s). Emulsifiers may be nonionic, anionic or cationic. Suitable emulsifiers are disclosed in, for example, CTFA Handbook, pp.1673-1686.
Lotions and creams can be formulated as emulsions. Typically such lotions comprise from 0.5% to 5% of an emulsifier(s). Such creams would typically comprise from 1% to 20% (e.g., from 5% to 10%) of an emollient(s); from 20% to 80% (e.g., from 30% to 70%) of water; and from 1% to 10% (e.g., from 2% to 5%) of an emulsifier(s).
Single emulsion skin care preparations, such as lotions and creams, of the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.
The topical compositions of this invention can also be formulated as a gel (e.g., an aqueous gel using a suitable gelling agent(s)). Suitable gelling agents for aqueous gels include, natural gums, acrylic acid and acrylate polymers and copolymers, and cellulose derivatives (e.g., hydroxymethyl cellulose and hydroxypropyl cellulose). Suitable gelling agents for oils (such as mineral oil) include, hydrogenated butylene/ethylene/styrene copolymer and hydrogenated ethylene/propylene/styrene copolymer. Such gels typically comprise between 0.1% and 5%, by weight, of such gelling agents.
In one embodiment, the composition is anhydrous. In one embodiment, such anhydrous composition is exothermic upon application.
The topical compositions of the present invention can also be formulated into a solid formulation (e.g., a wax-based stick, soap bar composition, powder, a wipe containing powder, or a dressing).
The topical compositions useful in the subject invention may contain, in addition to the aforementioned components, a wide variety of additional oil-soluble materials and/or water-soluble materials conventionally used in compositions for use on the skin at their art-established levels.
The topical compositions may be applied as needed and/or as part of a regular regimen ranging from application once a week up to one or more times a day (e.g., twice a day). The amount used will vary with the age and physical condition of the end user, the duration of the treatment, the specific compound, product, or composition employed, the particular cosmetically-acceptable carrier utilized, and like factors.

Several examples are described below.

### Example 1

A composition of the present invention (pH = 3.7) was prepared by first solubilizing salicylic acid and ethanol and then adding the remaining materials listed in Table 1 and mixing the materials in a homogenizer until homogenous.

**Table 1**

| **Ingredient** | **%W/W** |
|---|---|
| Ethanol | 40 |
| Salicylic Acid | 2 |
| C12-C15 alkyl lactate | 2 |
| Fragrance | 0.1 |
| Benzalkonium Chloride, 50% Solution USP | 0.2 |
| Aminomethyl Propanol | 0.19 |
| DI Water | Qs to 100% |

### Example 2

A composition of the present invention (pH = 3.7) was prepared in a similar manner as Example 1.

**Table 2**

| **Ingredient** | **%W/W** |
|---|---|
| Ethanol | 40 |
| Salicylic Acid | 2 |
| C₁₂-C₁₅ alkyl lactate | 2 |
| Fragrance | 0.1 |
| Benzalkonium Chloride, 50% Solution, USP | 0.2 |
| Glycerin, | 1 |
| Sodium Coco PG-Dimonium Chloride Phosphate | 0.06 |
| Aminomethyl Propanol | 0.19 |
| DI Water | Qs to 100% |

### Example 3

A composition (pH = 3.7) of the present invention was prepared by combining the materials listed in Table 3 as follows. First, the First Pre-mix ingredients were mixed and then the mixture was mixed with the Phase B ingredients until homogenous. The ingredients of phase A were also mixed until homogenous. Then, the phase B mixture was added and mixed into the phase B until uniform. The post addition ingredients and the second Pre-mix were then added to the resulting emulsion until uniform.

**Table 3**

| **Ingredient** | **%W/W** |
|---|---|
| First Pre-mix | |
| Phenethyl Dimethicone | 0.222 |
| Stearoxytrimethylsilane and Stearyl Alcohol | 0.028 |
| Ethanol | 4 |
| Second Pre-mix | |
| Feverfew Extract | 1 |
| DI water | 5 |
| Phase A | |
| Ethanol | 36 |
| Salicylic Acid | 2 |
| DI Water | 42.739 |
| Hydroxypropylcellulose | 1.5 |
| Glycerin | 3 |
| Phase B | |
| Cyclomethicone | 1.665 |
| C₁₂₋₁₅ alkyl lactate | 1 |
| Cetyl lactate | 0.5 |
| Propylene Glycol | 0.1 |
| Fragrance | 0.1 |
| Diazolidinyl Urea and Iodopropynyl Butvlcarbamate | 0.1 |
| Ethylene Glycol Phenyl Ether | 0.5 |
| Post Addition | |
| Benzalkonium Chloride, 50% Solution, USP | 0.2 |
| Sodium Coco PG-Dimonium Chloride Phosphate | 0.056 |
| Propylene Glycol and Diazolidinyl Urea and Methylparaben and Propylparaben | 0.1 |
| Aminomethyl Propanol | 0.19 |

### Example 4

A composition (pH = 3.7) of the present invention was prepared by combining the materials listed in Table 4 in the same manner as described in Example 3.

**Table 4**

| **Ingredient** | **%W/W** |
|---|---|
| Pre-mix | |
| Ethanol | 4 |
| Phenethyl Dimethicone | 0.2 |
| Stearoxytrimethylsilane and Stearyl Alcohol | 0.028 |
| | |
| Phase A | |
| Ethanol | 36 |
| Salicylic Acid | 2 |
| DI Water | 39.736 |
| Hydroxypropylcellulose | 1.5 |
| Glycerin | 3 |
| Phase B | |
| Cyclomethicone | 1.66 |
| C₁₂₋₁₅ alkyl lactate | 1 |
| Cetyl lactate | |
| Propylene Glycol | 0.1 |
| Fragrance | 0.13 |
| DI Water | 9 |
| Diazolidinyl Urea and Iodopropynyl Butylcarbamate | 0.1 |
| Ethylene Glycol Phenyl Ether | 0.5 |
| Post Addition | |
| Benzalkonium Chloride, 50% Solution, USP | 0.2 |
| Sodium Coco PG-Dimoniun 0.056 | 0.056 |

| Chloride Phosphate | |
|---|---|
| Propylene Glycol and Diazolidinyl Urea and Methylparaben and Propylparaben | 0.1 |
| Aminomethvl Propanol | 0.19 |

### Example 5

A composition (pH = 3.7) of the present invention was prepared by combining the materials listed in Table 5. For phase B, the water was heated to ~40oC and all other ingredients were added and mixed until dissolved. Phase A ingredients were mixed until homogenous. Phase A was added to phase B and mixed until uniform. Post addition ingredients were then added.

**Table 5**

| **Ingredient** | **%W/W** |
|---|---|
| Phase A | |
| C₁₂₋₁₅ alkyl lactate | 1 |
| Salicylic Acid | 0.5 |
| PEG-32 | 12 |
| Phase B | |
| DI water | 60 |
| glycerin | 2 |
| Methylethylcellulose | 0.75 |
| Isohexadecane and Ammonium Polyacryloylldimethyl Taurate and Polysorbate 80 | 1 |
| Post Addition | |
| Benzalkonium Chloride, 50% Solution, USP | 0.2 |
| Sodium Coco PG-Dimonium Chloride Phosphate | 1 |
| DI water | Qs to 100 |

### Example 6

A clinical study was conducted using the composition of Example 4 to treat mild to moderate acne. The study demonstrated the ability of the compositions of the present invention to quickly treat acne (twice a day product applications on the affected acne areas). A control composition of Clearasil® Acne Treatment Cream (Boots International Inc., London UK) containing 10% benzoyl peroxide ("10% BPO") and an untreated acne population receiving no treatment ("Untreated") were also used in the study. The acne severity was graded according to the following scale (as recommended by American academy of dermatology):

| no lesion | very slight | Slight | Mild | moderate | severe |
|---|---|---|---|---|---|
| 0.0 | 1.0 | 2.0 | 3.0 | 4.0 | 5.0 |

The results of the clinical study for mild to severe patients (baseline grade 3-5) are shown below in Table 6.

**Table 6**

| 48 hours | Sample | OVERALL | SIZE | REDNESS | ELEVATION | PEELING |
|---|---|---|---|---|---|---|
| Average | 10% BPO(n=5) | -0.2 | 0 | -0.4 | -0.6 | 1 |
| %improve | 10% BPO | 20 | 20 | 60 | 60 | 0 |
| %NC | 10% BPO | 80 | 60 | 20 | 40 | 40 |
| %worse | 10% BPO | 0 | 20 | 20 | 0 | 60 |
| Average | Example 4 (n=10) | -0.8 | -0.5 | -0.5 | -0.6 | 0.3 |
| %improve | Example 4 | 40 | 50 | 40 | 50 | 10 |
| %NC | Example 4 | 40 | 30 | 40 | 30 | 60 |
| %worse | Example 4 | 20 | 20 | 20 | 20 | 30 |
| Average | Untreated (n=2) | -0.5 | 0.5 | 0.5 | 1 | 0 |
| %improve | Untreated | 50 | 0 | 0 | 0 | 0 |
| %NC | Untreated | 50 | 50 | 50 | 50 | 100 |
| %worse | Untreated | 0 | 50 | 50 | 50 | 0 |

| | | | | | | |
|---|---|---|---|---|---|---|
| NC = no change | | | | | | |

The relative improvement to baseline in pimple size, redness and elevation for the mild acne lesions was superior for the formulation of Example 4 versus the 10% BPO treatment. There was also less peeling associated with the formulation of the present invention.

The results of the clinical study for moderate to severe acne patients whose pimples are more deeply rooted (baseline grade 4-5) are shown below in Table 7. Example 4 displayed surprising superior efficacy to treat all of the measured symptoms of moderate to severe acne as compared to untreated control and the 10% BPO cream.

**Table 7**

| 48 HRS | Sample | OVERAL L | SIZE | REDNES S | ELEVATIO N | PEELING |
|---|---|---|---|---|---|---|
| Average | 10% BPO n=3 | -0.3 | 0 | 0 | -0.3 | 1.3 |
| %improve | 10% BPO | 33.3 | 33.3 | 33.3 | 33.3 | 0 |
| %NC | 10% BPO | 66.7 | 33.3 | 33.3 | 66.7 | 33.3 |
| %worse | 10% BPO | 0 | 33.3 | 33.3 | 0 | 66.7 |
| Average | Example 4 (n=4) | -1.8 | -1.8 | -1.5 | -1.8 | -0.3 |
| %improve | Example 4 | 50 | 100 | 75 | 75 | 25 |
| %NC | Example 4 | 50 | 0 | 25 | 25 | 75 |
| %worse | Example 4 | 0 | 0 | 0 | 0 | 0 |
| Average | Untreated (n=2) | -0.5 | 0.5 | 0.5 | 1 | 0 |
| %improve | Untreated | 50 | 0 | 0 | 3 | 0 |
| %NC | Untreated | 50 | 50 | 50 | 50 | 100 |
| %worse | Untreated | 0 | 50 | 50 | 50 | 0 |

For moderate to severe acne, where pimples are often normally more deeply rooted, the composition of the present invention showed dramatic improvement in size, redness, elevation and peeling without irritating the skin, dryness, and forming noticeable scars and postmarks afterwards.

### Example 7

A clinical study was also conducted to evaluate the fast-acting efficacy of the formula of Example 4 versus Clearasil® Acne Treatment Cream in the treatment of mild to moderate acne vulgaris over a 1 week period. This study was a double blind study involving twice a day full-face application. Subjects were males and females between the ages of 12-30 with a Fitzpatrick Skin Type of I - IV. Subjects exhibited mild to moderate acne vulgaris at the baseline visit, with 1-3 target inflammatory lesions in the active phase.

Dermatologist clinical evaluations of the target lesion surprisingly showed that there was a statistically significant reduction in the erythema of the acne target lesions treated with the formulation of Example 4 as early as the four hour time point, with significant reductions in the diameter of the target lesions starting at eight hours, and a significant reduction in lesion height/elevation at Day 2, which were all maintained over time. BPO 10% had no significant effect at 4 hours, and there was a statistically significant difference between Example 4 and 10% BPO treatment cream in target lesion erythema at the 8-hour time point and in diameter at the week 1 time point, in favor of the current invention. Subjects using Example 4 also did not exhibit an increase in any of the skin irritation parameters (peeling, edema, erythema, burning, stinging, itching), as graded by the dermatologist or by the subjects. There was a statistically significant decrease in peeling and oiliness as evaluated by the dermatologist, and a significant decrease in itching and tightness as evaluated by the subjects at various time points versus baseline.

Subjects using Example 4 experienced significantly less peeling beginning at 4 hours and significantly less oiliness and dryness beginning at Day 1 versus those subjects using the 10% BPO treatment, which continued through the study. In contrast, subjects using the 10% BPO product experienced a statistically significant increase in oiliness at the Day 2 time point.

### Example 8

Two formulations containing 0.10% histamine were prepared according to the following tables:

**Table 8a**

| **Ingredient** | **% (wt/wt)** |
|---|---|
| Ethanol | 40 |
| Salicylic acid | 2 |
| DI water | 40 |
| Benzalkonium Chloride | 0.1 |
| Sodium Hydroxide | 0.27 |
| Histamine | 0.1 |
| DI water | 17.53 |

**Table 8b**

| **Ingredient** | **% (wt/wt)** |
|---|---|
| Ethanol | 40 |
| Salicylic acid | 2 |
| C₁₂₋₁₅ Alkyl lactate | 5 |
| DI water | 40 |
| Benzalkonium Chloride | 0.1 |
| Sodium Hydroxide | 0.27 |
| Histamine | 0.1 |
| DI water | 12.53 |

A male subject was recruited to evaluate the in-vivo delivery of histamine by these two formulations. Both formulations were shook and 0.05 ml of the solutions were withdrawn and spread onto pre-marked areas of equal size on different sides of the subject nose. The sensation and appearance were recorded. Typical histamine effects of erythema, itching, slight burning was observed for both formulas. The formula of Table 8b (containing the C12-15 alkyl lactate) had such visible response in less than 2 minutes and peaked at around 5 minutes. The formula of Table 8a (not containing the C12-15 alkyl lactate) started to experience such visible histamine effect only after about 5 minutes and peaked at around 10-15 minutes. The formula of Table 8b induced and itching sensation that lasted for than one hour, much longer than the formula of Table 8a's response of less than 35-40 minutes.

### Example 9

A composition was prepared using the following components in Table 9a:

**Table 9a**

| **Chemical Name** | **% wt** |
|---|---|
| Ethyl Alcohol | 39.3 |
| Salicylic Acid | 2 |
| Witch Hazel (Hamamelis Virginiana) & Ethanol | 5 |
| Hydroxyethylcellulose | 0.1 |
| Deionized Water | 35.804 |
| Glycerin | 3 |
| Phenoxyethanol | 0.555 |
| Benzalkonium Chloride, 50% Solution, USP | 0.2 |
| Propylene Glycol | 0.111 |
| Dipropylene Glycol Isocetheth-20 Acetate | 0.5 |
| Cyclopentasiloxane | 1.665 |
| Poly Sorbate 20 | 0.367 |
| Fragrance | 0.13 |
| Phenethyl Dimethicone | 0.222 |
| C12-15 Alkyl Lactate | 1.111 |
| Cetyl Lactate | 0.555 |
| Sodium Coco PG-Dimonium Chloride Phosphate | 1 |
| Polyacrylamide and C13-14 Isoparafin and Laureth 7 | 7.5 |
| Sodium Hydroxide (20% solution) | 0.88 |

The composition was prepared as follows: First, beaker A was charged with Dipropylene Glycol Isocetheth-20 Acetate and heated to 40C until it melted. In beaker B, the ethyl alcohol and salicylic acid were added and mixed. In beaker C, the deionized water and hydroxyethylcellulose were added, and the resultant mixture was stirred and heated to 35-40°C until uniform. The composition was then allowed to cool to room temperature. Once cooled, the contents from beaker B were added to beaker C and stirred until homogeneous. While continuing to mix, the following ingredients were added in order: witch hazel, glycerin, phenoxyethanol, benzalkonium chloride, and propylene glycol.

The Cyclopentasiloxane, Poly Sorbate 20, Phenethyl Dimethicone, fragrance, C12-15 alkyl lactate, cetyl lactate, and Sodium Coco PG-Dimonium Chloride Phosphate were then added to beaker A. The contents of beaker A were then added to beaker C, and the resulting composition was mixed until uniform. The pH of the uniform composition was then adjusted to be between 3.5-4.0 with the solution of 20% sodium hydroxide. The polyacrylamide and C13-14 Isoparaffin and Laureth 7 mixture was then added, with the batch mixed until homogeneous. The finished product was then packaged into 1-ounce tubes.

The 1-ounce tubes were distributed to males and females ages 12 to 30 years with Fitzpatrick Skin Types of I - IV. Subjects were instructed to every morning and evening to wash his/her face with the PURPOSE® Gentle Cleansing Wash (Johnson & Johnson Consumer Products Company, Skillman, NJ). They were instructed to squeeze approximately a nickel-sized amount of finished product onto their fingertips and apply it to their entire facial area, avoiding the eye, lip, and mouth areas. After each product application, they were instructed to not wash their face for at least three (3) hours after applying the test product.

A self-assessment questionnaire was prepared for the subjects to complete at the beginning of the study, weeks 1, 2, 5 and 6. Table 9b includes the condition and subjects perception of the condition at such time point. The grading scale was from 1-10, where 1 indicated the worst and 10 indicated the best. All results were normalized to Week 0, so that each number below indicates the change from baseline. A positive number indicates improvement.

**Table 10b**

| Indication | Week 0 | Week 1 | Week 2 | Week 5 | Week 6 |
|---|---|---|---|---|---|
| Pain/soreness associated with pimples | 0 | 0.67 | 0.78 | 0.30 | 0.40 |
| Pain/soreness associated with new pimples coming up but that can't be seen | 0 | 0.89 | 0.78 | 1.10 | 1.00 |
| Oiliness of skin | 0 | 0.72 | 2.50 | 2.25 | 2.20 |
| Shininess of skin | 0 | 0.06 | 1.67 | 1.15 | 0.45 |
| Pore size | 0 | 1.11 | 2.39 | 1.95 | 2.85 |
| Visibility of marks/scars from previous pimples | 0 | 0.89 | 1.61 | 1.75 | 2.05 |
| Skin smoothness | 0 | 0.89 1 | 2.11 | 1.95 | 2.25 |
| Dryness of skin | 0 | 0.67 1 | 0.94 | 1.20 | 1.25 |
| Flakiness of skin | 0 | 1.28 | 1.94 | 1.85 | 2.00 |

A benefit assessment questionnaire was also prepared for the subjects to be completed simultaneously with the self-assessment questionnaire. Table 10c includes the benefit and the subject's perception of the condition at the time point listed. The grading scale was from 1-5, where 1 indicates that the subject completely disagreed while 5 indicates that the subject completely agreed. The percentage of subjects rating the benefit statement as 4 (agree somewhat) or a 5 (agree completely) is shown in Table 10c below.

**Table 10c**

| Benefit | Week 1 | Week 2 | Week 5 | Week 6 |
|---|---|---|---|---|
| I felt new pimples starting to appear but they never appeared | 70 | 66.7 | 72.7 | 80 |
| My pimples are less sore/painful/irritated | 88.9 | 71.4 | 66.7 | 77.8 |
| My pores look less noticeable | 55.6 | 54.5 | 78.6 | 75 |
| My skin feels less oily | 66.7 | 75 | 100 | 83.3 |
| My skin feels less shiny | 54.5 | 63.6 | 100 | 69.2 |
| My skin feels smoother | 69.2 | 57.1 | 92.9 | 71.4 |
| My dark marks look less visible | 33.3 | 66.7 | 57.1 | 69.2 |
| This product stops pimples before they become visible | 76.9 | 71.4 | 93.3 | 76.5 |

Expert grading of global acne parameters were also completed at the beginning and at 2, 5, and 6 weeks. Expert grading took place in single room where lighting conditions were consistent throughout the study. The study was double-blind, so neither the grader nor the subject knew what treatment he/she was applying. Severity was graded from 0-4, with 0 indicating no acne or least severe and 4 indicating most severe. A decrease in score over time indicates improvement. The results are shown in Table 10d.

**Table 10d**

| | Week 0 | Week 2 | Week 5 | Week 6 |
|---|---|---|---|---|
| Severity | 2.23 | 1.94 | 1.73 | 1.70 |

## Claims

1. A composition comprising an anti-acne agent, an antimicrobial agent, and a lactate, wherein the anti-acne agent is salicylic acid, the lactate is selected from the group consisting of C₁₂-C₁₆ alkyl lactates and combinations thereof and the antimicrobial agent is selected from the group consisting of benzalkonium chloride, benzethonium chloride, cetylpyridinium chloride, 3-iodo-2-propynyl-N-butylcarbamate, hexetidine (5-amino-1, 3-bis-(2-ethylhexyl)-5-methyl-hexahydropyrimidine), Quatemium 15, triclosan, chlorhexidine digluconate, and combinations thereof.

2. The composition according to claim 1 wherein said composition further comprises a phospholipid.

3. The composition according to claim 2 wherein said phospholipid is sodium coco PG-Dimonium Chloride phosphate, cocamidopropyl PG-Dimonium Chloride Phosphate or myristamidopropyl PG-Dimonium Chloride phosphate.

4. A composition according to any one of claims 1-3 for use in a method of treating a follicular disease comprising applying to an area of skin in need of such treatment the composition.

5. The composition for the use according to claim 4, wherein said follicular disease is acne, rosacea, or seborrhea.

6. The composition for the use according to claim 5, wherein said method of treating acne comprises treating pre-emergent pimples.

7. The composition for the use according to claim 6, wherein said method of treating acne comprises treating blackheads.

8. The composition for the use according to claim 7, wherein the appearance of said acne is reduced within about eight hours.

9. A composition according to any one of claims 1-3 for use in a method of reducing the appearance of pores or oil on the skin comprising applying to an area of skin in need of such treatment the composition.

10. A composition according to any one of claims 1-3 for use in a method of evening skin tone or smoothing skin comprising applying to an area of skin in need of such treatment the composition.

11. The composition for the use according to claim 10 wherein said skin has freckles, post-inflammatory hyperpigmentation (PIH), or scars.

## Patentansprüche

1. Zusammensetzung, umfassend ein Anti-Akne-Mittel, ein antimikrobielles Mittel und ein Lactat, wobei das Anti-Akne-Mittel Salicylsäure ist, das Lactat ausgewählt ist aus der Gruppe bestehend aus C₁₂-C₁₆-Alkyllactaten und Kombinationen davon und das antimikrobielle Mittel ausgewählt ist aus der Gruppe bestehend aus Benzalkoniumchlorid, Benzethoniumchlorid, Cetylpyridiniumchlorid, 3-Iod-2-propinyl-N-butylcarbamat, Hexidin (5-Amino-1,3-bis(2-ethylhexyl)-5-methylhexahydropyridin), Quaternium 15, Triclosan, Chlorhexidindigluconat und Kombinationen davon.

2. Zusammensetzung gemäß Anspruch 1, wobei die Zusammensetzung ferner ein Phospholipid umfasst.

3. Zusammensetzung gemäß Anspruch 2, wobei das Phospholipid Natriumcoco-PGdimoniumchloridphosphat, Cocamidopropyl-PGdimoniumchloridphosphat oder Myristamidopropyl-PGdimoniumchloridphosphat ist.

4. Zusammensetzung gemäß einem der Ansprüche 1-3 für die Verwendung bei einem Verfahren zum Behandeln einer Follikelerkrankung, umfassend Aufbringen der Zusammensetzung auf einen Hautbereich mit Bedarf an einer derartigen Behandlung.

5. Zusammensetzung für die Verwendung gemäß Anspruch 4, wobei die Follikelerkrankung Akne, Rosazea oder Seborrhö ist.

6. Zusammensetzung für die Verwendung gemäß Anspruch 5, wobei das Verfahren zum Behandeln von Akne Behandeln von preemergenten Pickeln umfasst.

7. Zusammensetzung für die Verwendung gemäß Anspruch 6, wobei das Verfahren zum Behandeln von Akne Behandeln von Komedonen umfasst.

8. Zusammensetzung für die Verwendung gemäß Anspruch 7, wobei die Erscheinung der Akne innerhalb von etwa acht Stunden verringert wird.

9. Zusammensetzung gemäß einem der Ansprüche 1-3 für die Verwendung bei einem Verfahren zum Verringern des Erscheinens von Poren oder von Öl auf der Haut, umfassend Aufbringen der Zusammensetzung auf einen Hautbereich mit Bedarf an einer derartigen Behandlung.

10. Zusammensetzung gemäß einem der Ansprüche 1-3 für die Verwendung bei einem Verfahren zum Ausgleichen von Hauttönung oder Glätten von Haut, umfassend Aufbringen der Zusammensetzung auf einen Hautbereich mit Bedarf an einer derartigen Behandlung.

11. Zusammensetzung für die Verwendung gemäß Anspruch 10, wobei die Haut Sommersprossen, Nachentzündungs-Hyperpigmentation (PIH) oder Narben aufweist.

## Revendications

1. Composition comprenant un agent anti-acné, un agent antimicrobien et un lactate, où l'agent anti-acné est l'acide salicylique, le lactate est choisi dans le groupe constitué par les lactates d'alkyle en C₁₂-C₁₆ et leurs combinaisons et l'agent antimicrobien est choisi dans le groupe constitué par le chlorure de benzalkonium, le chlorure de benzéthonium, le chlorure de cétylpyridinium, le carbamate de 3-iodo-2-propynyl-N-butyle, l'hexétidine (5-amino-1,3-bis-(2-éthylhexyl)-5-méthyl-hexahydropyrimidine), le Quaternium 15, le triclosane, le digluconate de chlorhexidine, et leurs combinaisons.

2. Composition selon la revendication 1, où ladite composition comprend en outre un phospholipide.

3. Composition selon la revendication 2, où ledit phospholipide est le chlorophosphate de sodium-coco-PG-dimonium, le chlorophosphate de cocamidopropyl-PG-dimonium ou le chlorophosphate de myristamidopropyl-PG-dimonium.

4. Composition selon l'une quelconque des revendications 1 à 3 pour utilisation dans une méthode de traitement d'une maladie folliculaire comprenant l'application de la composition à une zone de peau nécessitant un tel traitement.

5. Composition pour utilisation selon la revendication 4, où ladite maladie folliculaire est l'acné, l'acné rosacée ou la dermatite séborrhéique.

6. Composition pour utilisation selon la revendication 5, où ladite méthode de traitement de l'acné comprend le traitement de pustules pré-émergents.

7. Composition pour utilisation selon la revendication 6, où ladite méthode de traitement de l'acné comprend le traitement des points noirs.

8. Composition pour utilisation selon la revendication 7, où l'apparition de ladite acné est réduite en environ huit heures.

9. Composition selon l'une quelconque des revendications 1 à 3 pour utilisation dans une méthode de réduction de l'apparition de pores ou d'huile à la surface de la peau comprenant l'application de la composition à une zone de peau nécessitant un tel traitement.

10. Composition selon l'une quelconque des revendications 1 à 3 pour utilisation dans une méthode d'égalisation de la teinte de la peau ou de lissage de la peau comprenant l'application de la composition à une zone de peau nécessitant un tel traitement.

11. Composition pour utilisation selon la revendication 10, où ladite peau a des taches de rousseur, une hyperpigmentation post-inflammatoire (HPI) ou des cicatrices.
